# EUROPÄISCHE PATENTSCHRIFT

(11) **EP 1 537 874 B1**
(45) Veröffentlichungstag und Bekanntmachung des Hinweises auf die Patenterteilung: **13.06.2007**
(21) Anmeldenummer: 03027833.7
(22) Anmeldetag: 04.12.2003
(51) Int. Cl.: A61K 36/16

(54) **Verfahren zur Herstellung von Extrakten des Ginkgo Biloba**
Method for preparing extracts from Ginkgo biloba
Procédé de production d'un extrait de Ginkgo biloba

(43) Veröffentlichungstag der Anmeldung: 08.06.2005
(73) Patentinhaber: Cognis IP Management GmbH, 40589 Düsseldorf (DE)
(72) Erfinder: Rull Prous, Santiago, 08034 Barcelona (ES); Alaoui Ismaili, Smail, 08290 Cerdanyola del Vallès (ES); Forner, Pedro, 08014 Barcelona (ES); Fabry, Bernd, Dr., 41352 Korschenbroich (DE)

(56) Entgegenhaltungen:
- EP-A- 0 431 536
- EP-A- 0 436 129
- WO-A-02/083158
- US-A- 5 700 468

## Beschreibung

### Gebiet der Erfindung

Die Erfindung befindet sich auf dem Gebiet der botanischen Extrakte und betrifft ein Verfahren zur Herstellung von Ginkgoextrakten mit verbesserter Reinheit.

### Stand der Technik

Der Ginkgobaum wurde schon von Darwin enthusiastisch als lebendes Fossil bezeichnet, weil alle seine Eigenschaften mit Langlebigkeit verbunden zu sein scheinen. Seit Jahrhunderten werden Ginkgos in China und Japan in Tempelgärten angepflanzt und gepflegt, andernfalls wäre diese Spezies gewiss längst ausgestorben. Ebenso lange werden Extrakte von Ginkgoblättern in der traditionellen Medizin Asiens eingesetzt. In China wird beispielsweise das sogenannte *"Bai-guo-ye*" zur Bekämpfung so unterschiedlicher Erkrankungen wie Atemwegsbeschwerden, Hörschwäche, hohen Blutdruck, Gedächtnisverlust, Angina pectoris, Hautausschlag und Magenschmerzen eingesetzt. Die westliche Medizin erkannte die Heilkraft der Ginkgoinhaltsstoffe erst in den 50er Jahre. Schwabe analysierte erstmals die Zusammensetzung und bestimmte die Aktivität der gefundenen Stoffe. Er war auch der erste, der einen 0,1 Gew.-%igen Ginkgoextrakt unter der Bezeichnung *Tebonin* in den Handel brachte. Heutige Produkte weise eine Verdünnung von 1 : 50 auf und sind unter den Handelsnamen *Kaveri, Tanakan, Rökan* oder *Ginkgold* bekannt. Die aktiven Inhaltsstoffe des Ginkgo sind vor allem Flavonoidglycoside ("Ginkgoflavonoide"), die u.a. (Iso)Quercitin, Kaempferol, Kaempferol-3-rhamnoside, Isorhamnetin, Luteolin, Luteolinglykoside, Sitosterolglycoside sowie hexacyclische Terpenlactone umfassen, die als Ginkgolide bzw. Bilobalide bezeichnet werden.

Auch die Herstellung von Ginkgoextrakten ist in der Literatur vielfach beschrieben worden. Gegenstand der EP 0360556 B1 (Indena) ist beispielsweise die Extraktion der Blätter mit einem Lösemittelgemisch aus einem Aromaten und einem niederen Alkohol. In der Druckschrift EP 0431536 B1 (Schwabe) wird ein mehrstufiges Verfahren beschrieben, bei dem Extraktion nacheinander mit Aceton oder niederen Alkoholen, Methylethylketon, Butanol oder Pentanol sowie schließlich Alkanen oder Cycloalkanen erfolgt. Weitere Verfahren sind beispielsweise in US 5,885,582 (Montana) oder ES 2036951 B1 (Euromed) beschrieben. Üblicherweise erfolgt nach der Extraktion die Trennung und Anreicherung der Wertstoffe über eine chromatographische Reinigung. Diese setzt, um ökonomisch arbeiten und Produkte mit Aktivsubstanzgehalten von mindestens 20 Gew.-% zur Verfügung stellen zu können, jedoch voraus, dass das Ausgangsmaterial mehr als 1 Gew.-% Wirkstoffe aufweist.

In den letzten Jahren ist jedoch zu beobachten, dass die im Markt befindlichen Ginkgo-Rohstoffe abnehmende Wirkstoffmengen aufweisen. Derzeit liegt der durchschnittliche Wirkstoffgehalt bei maximal 0,85 Gew.-% - entschieden zu wenig, um mit chromatographischen Methoden bei vertretbarem wirtschaftlichem Aufwand spezifikationsgerechte Extrakte herzustellen. Die Aufgabe der vorliegenden Erfindung hat somit darin bestanden, ein neues Verfahren zur Herstellung von Extrakten des *Ginkgo biloba* zur Verfügung zu stellen, bei dem ausgehend von Rohstoffen mit einem Wirkstoffgehalt (Ginkgoflavonglucoside, Ginkgoside, Bilobalide) von weniger als 1 Gew.-% Endprodukte mit Gehalten von wenigstens 20, vorzugsweise wenigstens 24 Gew.-% erhalten werden.

### Beschreibung der Erfindung

Gegenstand der Erfindung ist ein Verfahren zur Herstellung von Extrakten des *Ginkgo biloba* mit einem Gehalt an Ginkgoflavonglucosiden, Gingkoliden und Bilobaliden von insgesamt mindestens 20 Gew.-% aus Rohstoffen mit einem Gehalt an Ginkgoflavonglucosiden bezogen auf das Trockengewicht von weniger als 1 Gew.-%, welches sich dadurch auszeichnet, dass man die Ausgangsstoffe zunächst einer Extraktion mit wässrigem Aceton und dann einer mehrstufigen Flüssig-Extraktion mit (a) aliphatischen C₄-C₆-Alkoholen und/oder aliphatischen C₅-C₁₀ Kohlenwasserstoffen und (b) wässrigen Lösungsmitteln unterwirft.

Überraschenderweise wurde gefunden, dass die Kombination von Lösungsmittelextraktion und mehrstufiger Flüssig-Flüssig-Extraktion unter Einsatz der genannten Lösemittel auch ausgehend von wirkstoffarmen Rohmaterialien schließlich zu einem Extrakt führt, der einen Aktivsubstanzgehalt von deutlich über 20 Gew.-% aufweist.

### Durchführung des Verfahrens

Ein wesentliches Element des erfindungsgemäßen Verfahrens besteht in der Verwendung ausgewählter Lösemittel für die mehrstufige Flüssig-Flüssig-Extraktion, nämlich zum einen Wasser bzw. wässrige Alkalilauge und zum anderen organische Lösungsmittel vom Typ der aliphatischen C₄-C₆-Alkohole und/oder der aliphatischen C₅-C₁₀ Kohlenwasserstoffe.

In einer bevorzugten Ausführungsform der Erfindung erfolgt die Herstellung der Extrakte, indem man Blätter des *Ginkgo biloba*
(a) mit wässrigem Aceton extrahiert und eine organische Phase I erhält,
(b) die organische Phase I auf einen pH-Wert von 5 bis 6 einstellt, einer ersten Flüssig-Flüssig-Extraktion mit einem aliphatischen C₄-C₆-Alkohol unterwirft und dabei die organische Phase II erhält,
(c) die organische Phase II einer zweiten Flüssig-Flüssig-Extraktion mit Wasser unterwirft und dabei die organische Phase III erhält,
(d) die organische Phase III einer dritten Flüssig-Flüssig-Extraktion mit verdünnter wässriger Alkalilauge (pH = 7,5 bis 8,5) unterwirft und dabei die wässrige Phase IV gewinnt,
(e) die wässrige Phase IV einer abschließenden vierten Flüssig-Flüssig-Extraktion mit einem aliphatischen C₅-C₁₀ Kohlenwasserstoff unterwirft und dabei die wässrige Phase V gewinnt, und schließlich
(f) die wässrige Phase V in an sich bekannter Weise aufarbeitet und trocknet.

### Extraktion

Die Herstellung der Extrakte der ersten Stufe kann in an sich bekannter Weise erfolgen, d.h. beispielsweise durch wässrig-organischen Auszug der Pflanzen bzw. Pflanzenteile. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial wird vorzugsweise von Ginkgoblättern ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen wird wässriges Aceton eingesetzt, wobei die Mischung 35 bis 60 Gew.-% des organischen Lösemittels enthalten kann. Die Extraktion erfolgt in der Regel bei 25 bis 100 °C, bevorzugt bei 50 bis 70 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Im Anschluss an die Extraktion werden die Extrakte aufkonzentriert, bis nur noch maximal 5 Gew.-% Lösemittel vorhanden ist, unlösliche Bestandteile abfiltriert und mit 10 bis 15 Gew.-% Methanol versetzt. Das resultierende Produkt wird als "organische Phase I" bezeichnet.

### Flüssig-Flüssig-Extraktion

Die organische Phase I ist der Ausgangsstoff für die nachfolgende mehrstufige Flüssig-Flüssigextraktion, die bei mindestens 25 °C durchgeführt wird, wobei die Temperatur nach oben durch den Siedepunkt des jeweils verwendeten Lösemittels begrenzt wird. Die erste Stufe der Flüssig-Flüssig-Extraktion wird mit einem niederen aliphatischen Alkohol durchgeführt, wobei sich n-Butanol besonders bewährt hat. Es empfiehlt sich, die organische Phase I zuvor durch Zugabe von verdünnter Alkalilauge auf einen pH-Wert im Bereich von 4,5 bis 7 einzustellen. Das Zielprodukt dieser Stufe ist die organische Phase II, die in die nächste Extraktion eingesetzt wird, während die wässrige Phase verworfen werden kann. Die zweite Extraktion erfolgt mit Wasser, wobei wieder eine organische Wertphase III sowie eine wässrige Abfallphase erhalten wird. Die organische Phase wird dann in der dritten Stufe mit verdünnter wässriger Alkalilauge extrahiert, wobei die Wertstoffe sich jedoch diesmal in der wässrigen Phase IV wiederfinden, während die organische Abfallphase verworfen werden kann. Die wässrige Phase wird schließlich durch Zugabe von verdünnter Mineralsäure auf pH = 3,5 bis 5,5 eingestellt, bis auf einen Trockenrückstand von 10 bis 30 Gew.-% eingedampft, durch Filtration von unlöslichen Bestandteilen befreit und dann in die vierte und letzte Stufe der Flüssig-Flüssig-Extraktion geführt, die mit niederen aliphatischen Kohlenwasserstoffen, vorzugsweise n-Heptan betrieben wird. Die Wertstoffe befinden sich hier abermals in der wässrigen Phase V, während vor allem die unerwünschten Ginkgosäuren in die organische Abfallphase wandern. Die wässrige Phase kann sodann in an sich bekannter Weise aufgearbeitet werden, d.h. sie wird aufkonzentriert, mit Ethanol gewaschen, von unlöslichen Bestandteilen durch Filtration befreit und schließlich getrocknet, wobei dann schließlich ein Trockenextrakt gewonnen wird, der mindestens 20, vorzugsweise aber 24 bis 30 Gew.-% an Wertstoffen enthält.

### Beispiele

### Beispiel 1

In einem Rührkessel wurden 1000 g Blätter des *Ginkgo biloba* mit einem Gehalt an Ginkgoflavonglucosiden (GFG), Ginkgoliden und Bilobaliden von insgesamt 0,8 Gew.-% vorgelegt und bei 50 °C 2 h mit 5 l wässrigem Aceton (ca. 50 Gew.-%ig) extrahiert. Anschließend wurde das Lösungsmittel vom Rückstand abgetrennt und die so gewonnene organische Phase eingedampft, bis eine konzentrierte Flüssigkeit erhalten wurde, die noch 4 Gew.-% Aceton enthielt und einen Trockenrückstand von 25 Gew.-% aufwies. Nach Abtrennung der unlöslichen Rückstände wurde die konzentrierte Flüssigkeit mit 30 Gew.-% Methanol versetzt und mit verdünnter wässriger Alkalilauge auf einen pH-Wert von 5,5 eingestellt. Diese organische Phase I wurde dann einer dreistufigen Flüssig-Flüssig-Extraktion zunächst mit n-Butanol, dann mit Wasser und schließlich verdünnter wässriger Base unterworfen, wobei in den beiden ersten Schritten jeweils die organischen Phasen II und III, im letzten jedoch die wässrige Phase IV weiterverwendet wurde. Letztere, also das Produkt der alkalisch-wässrigen Extraktion wurde durch Zugabe von verdünnter Mineralsäure schwach sauer auf pH = 4,5 eingestellt, bis auf einen Trockenrückstand von 25 Gew.-% eingedampft und dann durch Filtration von unlöslichen Rückständen befreit. Die konzentrierte wässrige Phase wurde dann einer abschlie-ßenden Flüssig-Flüssig-Extraktion mit n-Heptan unterworfen, die als Wertprodukt eine wässrige Phase V lieferte. Diese wurde dann konzentriert, abermals von unlöslichen Bestandteilen befreit und getrocknet, wobei letztlich ein Extrakt erhalten wurde, der einen Gehalt an Aktivsubstanzen (GFG, Ginkgoliden und Bilobaliden) von rund 28 Gew.-% aufwies.

## Patentansprüche

1. Verfahren zur Herstellung von Extrakten des *Ginkgo biloba* mit einem Gehalt an Ginkgoflavonglucosiden, Gingkoliden und Bilobaliden von insgesamt mindestens 20 Gew.-% aus Rohstoffen mit einem Gehalt an Ginkgoflavonglucosiden bezogen auf das Trockengewicht von weniger als 1 Gew.-%, **dadurch gekennzeichnet, dass** man die Ausgangsstoffe zunächst einer Extraktion mit wässrigem Aceton und dann einer mehrstufigen Flüssig-Flüssig-Extraktion mit (a) aliphatischen C₄-C₆-Alkoholen und/oder aliphatischen C₅-C₁₀ Kohlenwasserstoffen und (b) wässrigen Lösungsmitteln unterwirft.

2. Verfahren nach Anspruch 1, **dadurch gekennzeichnet, dass** man Blätter des *Ginkgo biloba*
(a) mit wässrigem Aceton extrahiert und eine organische Phase I erhält,
(b) die organische Phase I auf einen pH-Wert von 5 bis 6 einstellt, einer ersten Flüssig-Flüssig-Extraktion mit einem aliphatischen C₄-C₆-Alkohol unterwirft und dabei die organische Phase II erhält,
(c) die organische Phase II einer zweiten Flüssig-Flüssig-Extraktion mit Wasser unterwirft und dabei die organische Phase III erhält,
(d) die organische Phase III einer dritten Flüssig-Flüssig-Extraktion mit verdünnter wässriger Alkalilauge (pH = 7,5 bis 8,5) unterwirft und dabei die wässrige Phase IV gewinnt,
(e) die wässrige Phase IV einer abschließenden vierten Flüssig-Flüssig-Extraktion mit einem aliphatischen C₅-C₁₀ Kohlenwasserstoff unterwirft und dabei die wässrige Phase V gewinnt, und schließlich
(f) die wässrige Phase V in an sich bekannter Weise aufarbeitet und trocknet.

3. Verfahren nach mindestens einem der Ansprüche 1 und/oder 2, **dadurch gekennzeichnet, dass** man als aliphatischen C₄-C₆-Alkohol n-Butanol einsetzt.

4. Verfahren nach mindestens einem der Ansprüche 1 bis 3, **dadurch gekennzeichnet, dass** man als aliphatischen C₅-C₁₀ Kohlenwasserstoffn-Heptan einsetzt.

5. Verfahren nach mindestens einem der Ansprüche 1 bis 4, **dadurch gekennzeichnet, dass** man die Extraktionen bei Temperaturen von mindestens 25°C durchführt, wobei sich die obere Grenze aus dem Siedepunkt des verwendeten Lösemittels ergibt.

## Claims

1. A process for the production of extracts of *Ginkgo biloba* containing a total of at least 20% by weight of ginkgo flavonglucosides, gingkolides and bilobalides from raw materials with a ginkgo flavonglucoside content, based on dry weight, of less than 1% by weight, **characterized in that** the starting materials are subjected first to extraction with aqueous acetone and then to a multistage liquid/liquid extraction with (a) aliphatic C₄₋₆ alcohols and/or aliphatic C₅₋₁₀ hydrocarbons and (b) aqueous solvents.

2. A process as claimed in claim 1, **characterized in that** leaves of *Ginkgo biloba* are
(a) extracted with aqueous acetone, an organic phase I being obtained,
(b) the organic phase is adjusted to a pH of 5 to 6 and subjected to a first liquid/liquid extraction with an aliphatic C₄₋₆ alcohol, an organic phase II being obtained,
(c) the organic phase II is subjected to a second liquid/liquid extraction with water, an organic phase III being obtained,
(d) the organic phase III is subjected to a third liquid/liquid extraction with dilute aqueous alkali metal hydroxide (pH = 7.5 to 8.5), an aqueous phase IV being obtained,
(e) the aqueous phase IV is subjected to a concluding fourth liquid/liquid extraction with an aliphatic C₅₋₁₀ hydrocarbon, an aqueous phase V being obtained, and finally
(f) the aqueous phase V is worked up and dried in known manner.

3. A process as claimed in at least one of claims 1 and/or 2, **characterized in that** n-butanol is used as the aliphatic C₄₋₆ alcohol.

4. A process as claimed in at least one of claims 1 to 3, **characterized in that** n-heptane is used as the aliphatic C₅₋₁₀ hydrocarbon.

5. A process as claimed in at least one of claims 1 to 4, **characterized in that** the extractions are carried out at temperatures of at least 25°C, the upper limit being determined by the boiling point of the solvent used.

## Revendications

1. Procédé de préparation d'extraits de *Ginkgo biloba* dont la teneur totale en glucosides flavonoïdes de ginkgo, en ginkgolides et en bilobalides est d'au moins 20 % au total, à partir de matières brutes ayant une teneur en glucosides flavonoïdes de ginkgo inférieure à 1 % en poids par rapport au poids sec,
**caractérisé en ce qu'**
on soumet les substances de départ d'abord à une extraction avec de l'acétone aqueuse, puis à une extraction liquide-liquide en plusieurs étapes avec (a) des alcools aliphatiques en C₄ à C₆ et/ou des hydrocarbures aliphatiques en C₅ à C₁₀ et (b) des solvants aqueux.

2. Procédé selon la revendication 1,
**caractérisé en ce qu'**
(a) on extrait des feuilles de *Ginkgo biloba* avec de l'acétone aqueuse pour obtenir une phase organique I,
(b) on règle le pH de la phase organique 1 à 5 à 6, on la soumet à une première extraction liquide-liquide avec un alcool aliphatique en C₄ à C₆ pour obtenir la phase organique II,
(c) on soumet la phase organique II à une deuxième extraction liquide-liquide avec de l'eau pour obtenir la phase organique III,
(d) on soumet la phase organique III à une troisième extraction liquide-liquide avec une lessive alcaline aqueuse diluée (pH = 7,5 à 8,5) pour obtenir une phase aqueuse IV,
(e) on soumet la phase aqueuse IV à une dernière et quatrième extraction liquide-liquide avec un hydrocarbure aliphatique en C₅ à C₁₀ pour obtenir une phase aqueuse V puis
(f) on purifie la phase aqueuse V d'une manière connue et on sèche.

3. Procédé selon au moins l'une des revendications 1 et/ou 2,
**caractérisé en ce qu'**
on utilise du n-butanol en tant qu'alcool aliphatique en C₄ à C₆.

4. Procédé selon au moins l'une des revendications 1 à 3,
**caractérisé en ce qu'**
on utilise de l'n-heptane en tant qu'hydrocarbure aliphatique en C₅ à C₁₀.

5. Procédé selon au moins l'une des revendications 1 à 4,
**caractérisé en ce qu'**
on met en oeuvre les extractions à des températures d'au moins 25 °C, la limite supérieure résultant du point d'ébullition du solvant utilisé.
